# EUROPEAN PATENT APPLICATION

(11) **EP 4 789 687 A1**
(43) Date of publication of application: **12.08.2026**
(21) Application number: 26151428.5
(22) Date of filing: 12.01.2026
(51) Int. Cl.: A61K 49/18, B82Y 15/00

(54) **SPION/HA-FA CONTRAST AGENT AND METHOD OF OBTAINING IT**

(30) Priority: 10.01.2025 PL 45093825
(71) Applicant: UNIWERSYTET JAGIELLONSKI, 31-007 Kraków (PL); Akademia Gorniczo-Hutnicza im. Stanislawa Staszica w Krakowie, 30-059 Krakow (PL)
(72) Inventor: Kasprzyk, Martyna, 31-509 Kraków (PL); Hinz, Alicja, 31-201 Kraków (PL); Karewicz, Anna, 31-272 Kraków (PL); Dulinska-Litewka, Joanna, 31-216 Kraków (PL); Kapusta, Czeslaw, 31-355 Kraków (PL); Opila, Gabriela, 30-611 Kraków (PL)
(74) Representative: Witek, Rafal

(57) **Abstract**

A new contrast agent (SPION/HA-FA) is disclosed, consisting of superparamagnetic iron oxide nanoparticles (SPION) surface-stabilized with low-molecular weight hyaluronic acid (HA) with covalently bound folic acid (FA) and the process for the preparation thereof. The contrast agent of the invention is particularly suitable for the imaging of neoplastic tumors using the magnetic resonance imaging (MRI) technique.

## Description

The subject matter of the invention is a new contrast agent (SPION/HA-FA) consisting of superparamagnetic iron oxide nanoparticles (SPION) surface-stabilized with low-molecular weight hyaluronic acid (HA) with covalently bound folic acid (FA). The superparamagnetic particles are characterized by temporal fluctuations of magnetic moments of respective particles that generate fluctuating magnetic fields which accelerate nuclear magnetic resonance relaxation in MRI.

Owing to the properties disclosed in the application, the agent is particularly suitable for the imaging of neoplastic tumors using the magnetic resonance imaging (MRI) technique.

Various substances are known as contrast agents specific against the neoplastic tissue and useful in the diagnosis and localization of neoplastic tumors of specific types.

Nevertheless, there is still a need for contrast agents with improved properties, in particular high stability both during storage and use, improved magnetic properties to achieve higher sensitivity in MRI examinations and shorter duration of the examination as well as having selective high specificity against specific types of neoplasms.

The objective of the invention has been to obtain such an improved contrast agent.

The subject matter of the invention is a contrast agent (SPION/HA-FA) consisting of superparamagnetic iron oxide nanoparticles (SPION) surface-stabilized with low-molecular weight hyaluronic acid (HA) with covalently attached folic acid (FA).

Preferably, the contrast agent of the invention has a form of nanoparticles with hydrodynamic diameter below 200 nm and showing stability in the physiological environment for at least 50 hours.

Another subject matter of the invention is the process for the preparation of the contrast agent, characterized in that:
a) superparamagnetic iron oxide nanoparticles stabilized with low-molecular weight hyaluronic acid (SPION/HA) are obtained,
b) the surface of SPION/HA obtained in step a) is modified by covalently attaching folic acid molecules, and subsequently
c) the superparamagnetic iron oxide nanoparticles surface-stabilized with low-molecular weight hyaluronic acid with covalently attached folic acid (SPION/HA-FA) obtained in step b) are separated.

Preferably, in step a), a solution of hyaluronic acid sodium salt in ammonia water is pretreated by stirring for at least 18 hours at room temperature and pulse sonication for 2.5-4 hours at not less than 50°C, subsequently the temperature of the solution is set at 60°C and in the presence of an inert gas, iron(III) chloride and iron(II) chloride are added, preferably in a molar ratio of 2:1, followed by admixing of an ammonia water solution, preferably in an amount that achieves a concentration in the reaction mixture in the range of 1.5-2.0 M, and then it is left to obtain a colloidal suspension of SPION/HA, which is separated.

Preferably, in step b), SPION/HA modified with cystamine dichloride (SPION/HA-CYS) is first obtained by conducting the SPION/HA reaction in the presence of N-(3-dimethylaminopropyl)-N'-ethylcarbodiimide (EDC) and N-hydroxysuccinimide (NHS), preferably in a 1:1 ratio, continuing the reaction for 15-24 hours at 37°C with shaking, and then the thus obtained SPION/HA-CYS is separated and reacted with folic acid dissolved in anhydrous dimethyl sulfoxide (DMSO) in the presence of EDC, NHS, preferably in a 1:1 ratio, and triethylamine (TEA) as a catalyst, without light, previously bringing the pH of the SPION/HA-CYS suspension to the range of 9.5-10.

Preferably, in step c) dialysis against deionized water is carried out, particularly preferably on a membrane having an MWCO of 14 kDa.

In the context of the invention, the term "nanoparticles" of iron oxide refers to the magnetic cores of SPION/HA and SPION/HA-FA systems, of a size in the range of 5-15 nm, subjected to a surface stabilization process with low-molecular weight hyaluronic acid (HA) and then covalent attachment of folic acid, which form stable objects in the aqueous environment with a hydrodynamic diameter of no more than 200 nm.

In the context of the invention, the term "superparamagnetic" particles means that they are characterized by temporal fluctuations of magnetic moments of respective particles that generate fluctuating magnetic fields which accelerate nuclear magnetic resonance relaxation in MRI.

In the context of the invention, the term "stabilized" means that the magnetic cores formed by the iron oxide nanoparticles are surrounded by a thin layer of hyaluronic acid which ensures their stability (no aggregation) in an aqueous suspension (colloidal suspension).

In the context of the invention, the term "low-molecular weight" hyaluronic acid means hyaluronic acid in the form of sodium hyaluronate having a mean molecular weight in the range of 30 to 50 kDa.

In order to better explain the gist of the invention, its embodiments are presented below, additionally illustrated in the attached drawings.

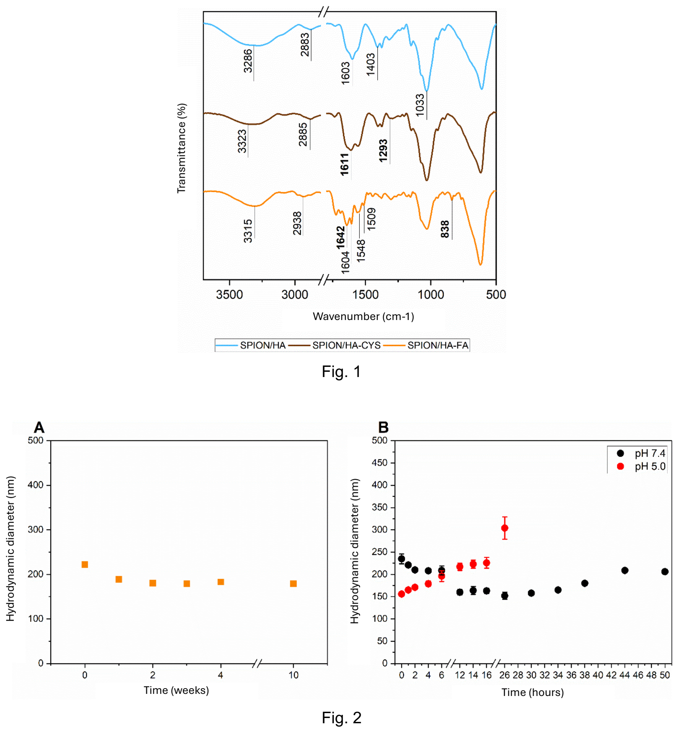

Figure 1 shows an ATR-FTIR spectrum of the obtained SPION coated with low-molecular weight hyaluronic acid (SPION/HA; blue line), SPION/HA with cystamine (SPION/HA-CYS; brown line) and SPION/HA with folic acid covalently coupled via cystamine (SPION/HA-FA; orange line).
Figure 2 shows in this order: A) long-term effect of storage time (refrigerator at 4°C) on the hydrodynamic diameter value of SPION/HA-FA and B) effect of incubation time on the hydrodynamic diameter value of SPION/HA-FA incubated in physiological conditions, i.e. at 37°C and at pH=7.4 or pH=5.0.

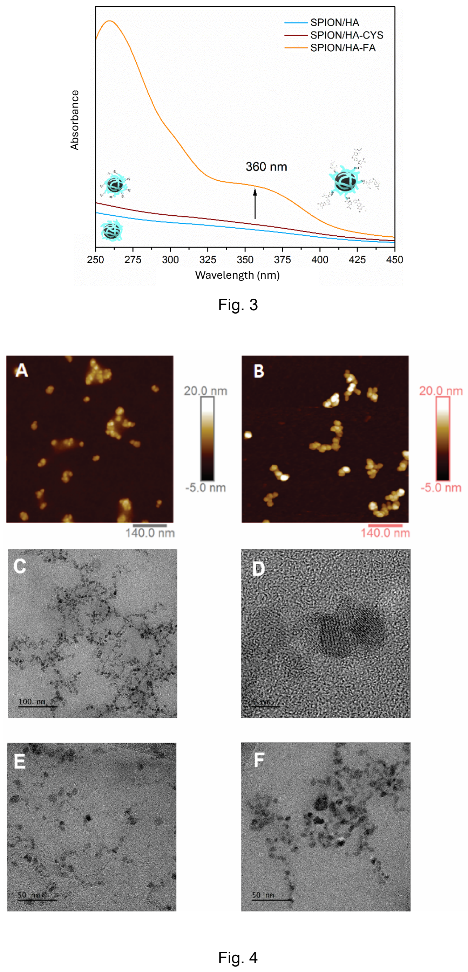

Figure 3 shows UV-VIS spectra of the obtained SPION/HA and their modifications with particular emphasis on the appearance of a maximum at λ = 360 nm (maximum absorption for aqueous folic acid solutions) for surface functionalization of SPION/HA with FA.
Figure 4 shows AFM images of: A) SPION/HA nanoparticles and B) SPION/HA-FA nanoparticles. TEM microphotographs of: C) SPION/HA-FA (x 64,000), D) SPION/HA-FA (x 930,000). Comparison of E) SPION/HA and F) SPION/HA-FA at the same magnification (x 130,000).

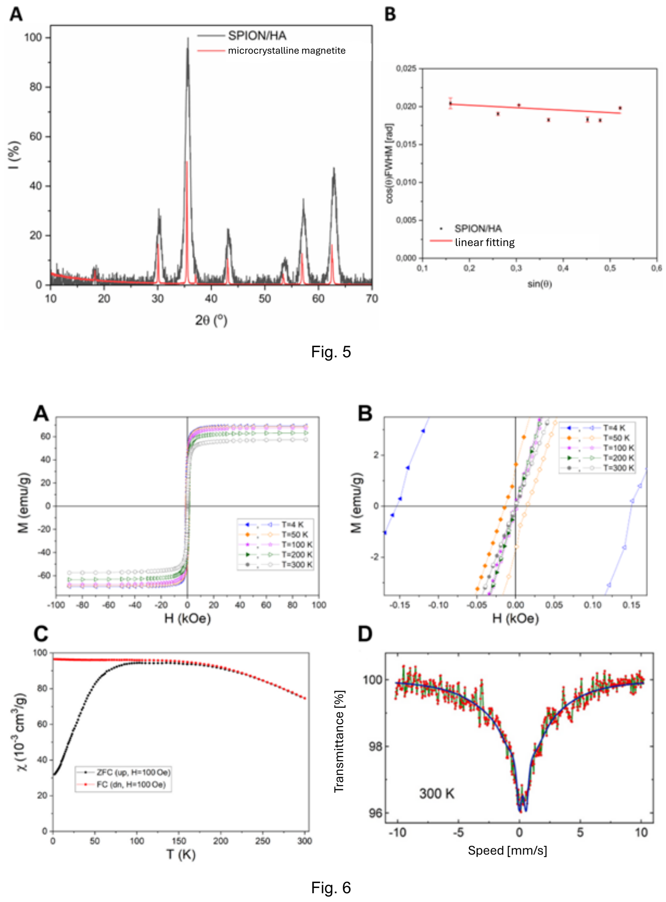

Figure 5 shows in this order: A) diffractogram of SPION/HA and microcrystalline magnetite and B) William-Hall graph for SPION/HA.
Figure 6 shows in this order: A) magnetization curves - hysteresis loops for selected temperatures, B) zoom of panel A) around zero, C) temperature dependence of magnetic susceptibility at 100 Oe - curves after cooling in zero field (ZFC) and in 100 Oe field (FC) and D) ⁵⁷Fe Moessbauer spectrum at room temperature.

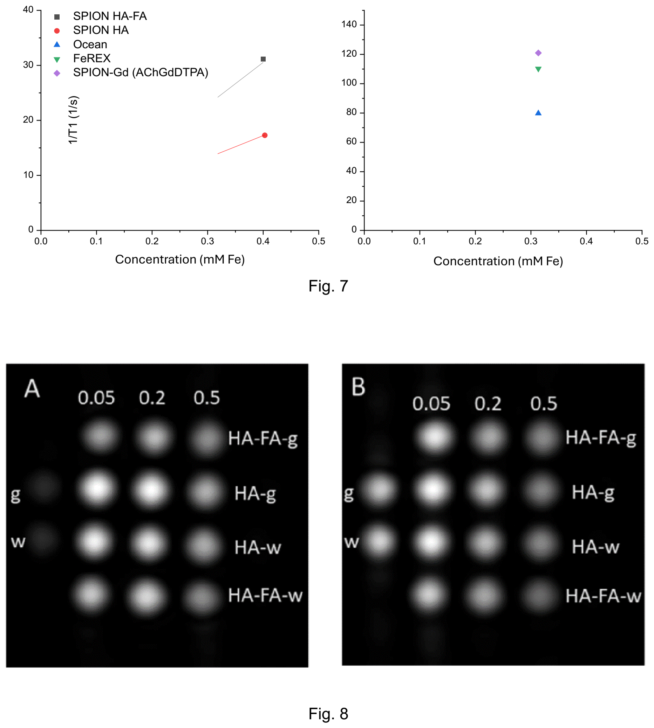

Figure 7 shows in this order: A) ¹H NMR 1/T₁ relaxation rate for SPION/HA and SPION/HA-FA as a function of concentration expressed in mM of Fe and B) ¹H NMR 1/T₂ relaxation rate for SPION/HA and SPION/HA-FA as a function of concentration expressed in mM of Fe. Literature data [A. Szpak et al., "T1-T2 Dual-modal MRI contrast agents based on superparamagnetic iron oxide nanoparticles with surface attached gadolinium complexes," Journal of Nanoparticle Research, vol. 16, no. 11, Nov. 2014, doi: 10.1007/s11051-014-2678-6] are added in both charts for comparison.
Figure 8 shows successively images: T₁ weighted (A), repetition time 0.5 s, echo time 7 ms and T₂ weighted (B), repetition time 10 s, echo time 7 ms, for MRI scans of a matrix of Eppendorf tubes containing different concentrations (in mM of Fe, see column labels) of SPION/HA and SPION/HA-FA aqueous suspensions (w) and gel phantoms (g), together with reference samples of water (w) and gel (g).

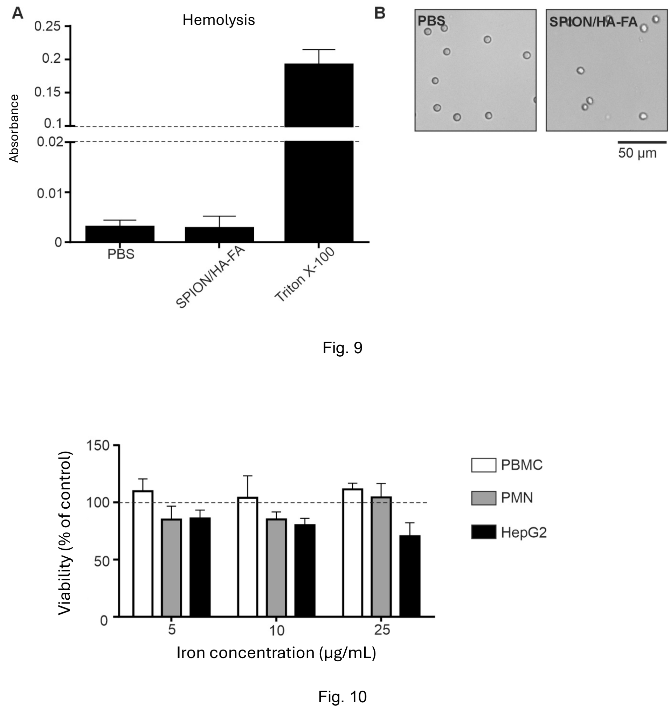

Figure 9 shows results of the analysis of the hemolytic activity of the tested nanoparticles. Human erythrocytes were incubated for 3 hours with PBS (negative hemolysis control), 0.4% Triton X-100 (positive hemolysis control) or SPION/HA-FA nanoparticles. Figure 9A shows the level of released hemoglobin determined spectrophotometrically after reaction with Drabkin's reagent. The bars represent means ± standard deviation of two independent experiments. Figure 9B shows erythrocyte morphology verified by bright field optical microscopy.
Figure 10 shows the effect of SPION/HA-FA interactions on the viability of human immune cells and hepatocytes (HepG2). Viability analysis was carried out after 24-hour incubation of the cells with nanoparticles: PBMC (mononuclear cells isolated from human peripheral blood), PMN (neutrophils isolated from human peripheral blood) analyzed in the ATPlite assay, and HepG2 (analyzed in the MTT assay). The bars show the mean viability of the negative control (100% viability) ± standard deviation of three independent experiments.

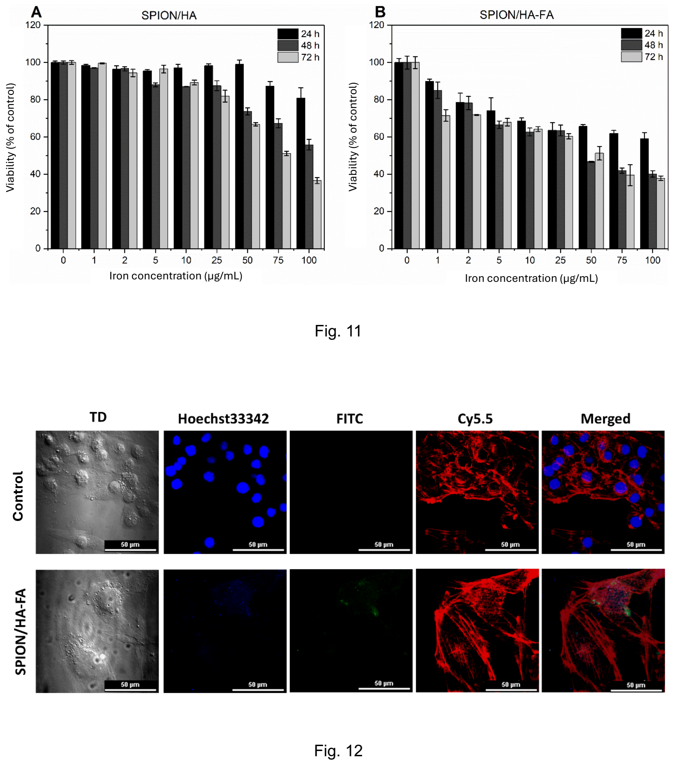

Figure 11 shows results of the MTT test for the human A172 glioblastoma multiforme line at 24, 48 and 72 hours after administration of A) SPION/HA and B) SPION/HA-FA.
Figure 12 shows images from a confocal laser scanning microscope of A172 cells incubated with fluorescein isothiocyanate (FITC) labeled SPION/HA-FA. Control: fluorescence image (40x lens) of control cells with stained cell nuclei (blue fluorescence) and F-actin (red fluorescence); SPION/HA-FA: fluorescence image (100x lens) of cells with stained F-actin (red fluorescence) after incubation with FITC-labeled SPION/HA-FA (blue fluorescence from FA and green fluorescence from FITC).

**Example 1.** Synthesis of the SPION/HA-FA contrast agent consisting of superparamagnetic iron oxide nanoparticles (SPION) surface-stabilized with low-molecular weight hyaluronic acid (HA) with covalently attached folic acid (FA).

### Preparation of SPION stabilized with low-molecular weight hyaluronic acid (SPION/HA)

To obtain SPION stabilized with low-molecular weight HA, 100 mg of hyaluronic acid sodium salt with a mean molecular weight of Mw = (30-50) kDa was weighed and dissolved in 9 mL deionized water at room temperature. 1 mL of 5 M ammonia water was added and the solution was stirred at room temperature for 18 hours. The solution was then pulsed sonicated (480 W, 1-second pulse every 5 seconds) for 3 hours at 50°C. The mixture was subsequently placed in a three-necked flask on a magnetic stirrer under constant flow of argon gas and heated at 60°C for 15 minutes. Hydrates of iron(III) and iron(II) chlorides were weighed in a molar ratio of 2:1 (23.2 mg FeCl₃·6H₂O and 8.5 mg FeCl₂·4H₂O) and each was dissolved in 0.5 mL deionized water and then mixed. 1 mL of the resulting solution was added dropwise to a vigorously mixed hyaluronic acid sodium salt solution, and another 4 mL of the 5 M ammonia water solution was then quickly added. The reaction was continued without changing the conditions for 30 minutes. The resulting colloidal suspension of nanoparticles was cooled to room temperature and purified through dialysis (14 kDa MWCO membrane) against deionized water for 24 hours, followed by magnetic chromatography (a glass Pasteur pipette was packed with steel wool, placed on a tripod between two neodymium magnets, moistened with deionized water and loaded with a suspension of resulting SPION/HA; the nanoparticles were eluted from the column using deionized water after removing it from the magnetic field). Finally, the colloid was filtered using a syringe filter with a 0.45 µm pore size polyether sulfone membrane. The product was kept at 4°C.

### Surface modifications of SPION/HA resulting in SPION/HA with covalently attached folic acid

Functionalization of SPION/HA with FA is a two-step process. SPION/HA were modified with cystamine dichloride (CYS) by adding 71.25 mg of N-(3-dimethylaminopropyl)-N'-ethylcarbodiimide hydrochloride (EDC) and 42.50 mg of N-hydroxysuccinimide (NHS) to 5 mL of the SPION/HA suspension. The suspension was shaken (350 rpm) on a laboratory shaker for 15 minutes at 37°C. 42.50 mg of CYS was then added and the reaction was continued in the same conditions for another 16 hours. The resulting SPION/HA-CYS were purified by dialysis (14 kDa MWCO membrane) against deionized water for 24 hours and concentrated by magnetic chromatography. The concentration of the introduced primary amine groups was estimated using the TNBS colorimetric method based on a calibration curve (R² = 0.9999; concentrations of amine groups in the range from 0.1 mM to 5.0 mM). The signal from unmodified SPION/HA was used as a reference.

20 mg of folic acid (FA) was then dissolved in 0.8 mL of anhydrous dimethyl sulfoxide (DMSO) and 43.43 mg of EDC, 26.07 mg of NHS and 100 µL of triethylamine (TEA) were added. The solution was stirred on a magnetic stirrer in the dark for 20 minutes. The resulting solution was added to 5 mL of previously obtained SPION/HA-CYS with pH = 10. pH was adjusted by adding 120 µL of 0.2 M sodium hydroxide solution to the colloid. The coupling reaction was carried out for 24 hours on a magnetic stirrer in the dark at room temperature. The resulting SPION/HA-FA was purified by dialysis (14 kDa MWCO membrane) against deionized water for 24 hours and then concentrated by magnetic chromatography.

To estimate the amount of FA attached to the surface of the nanoparticles, the suspension was centrifuged (20 minutes, 10,000 rpm, 4°C) and resuspended in deionized water in the volume initially used for synthesis. The resulting colloid was then diluted and its absorbance was measured at a wavelength of 360 nm. The amount of FA attached was determined on the basis of a calibration curve (R² = 0.9995; FA concentrations in the range of 13.8-69.0 µg/mL). The signal from the SPION/HA-CYS used for the FA coupling reaction was used as a reference. The resulting SPION/HA-CYS and SPION/HA-FA were stored at 4°C.

### Example 2. Physicochemical properties of the resulting nanoparticles

### Physicochemical characteristics of the resulting SPIONs

Based on dynamic light scattering (DLS) measurements, the value of the hydrodynamic diameter of fully swollen SPION/HA nanoparticles was determined, which was (137±2) nm. The measured polydispersity index was satisfactory and it was (0.258 ± 0.009), while the surface potential was equal to (- 53.0 ± 1.0) mV and indicated that the nanoparticles' suspension was colloidally stable. The presence of hyaluronic acid on the surface of SPION/HA was confirmed by infrared absorption spectroscopy (ATR-FTIR). The resulting spectrum had bands characteristic of hyaluronic acid (HA) at 3286 cm⁻¹ (-OH stretching vibrations of carboxyl groups), 2883 cm⁻¹ (-CH stretching vibrations), 1603 cm⁻¹ and 1403 cm⁻¹ (-COO antisymmetric and symmetric stretching vibrations). The spectrum is shown in Figure 1.

The presence of CYS on the surface of SPION/HA-CYS was also confirmed on the basis of the ATR-FTIR spectrum (*Figure 1*), where the characteristic bands were observed: N-H stretching vibrations of primary amines at 1611 cm⁻¹ and C-N stretching vibrations of primary amines at 1211 cm⁻¹. The amount of primary amino groups present in the SPION/HA-CYS suspension was evaluated spectrophotometrically using the TNBS method to be 2.2 ± 0.1) mM.

The nanoparticles after functionalization with folic acid (SPION/HA-FA) were characterized by an average hydrodynamic diameter of about (182±2) nm, a polydispersity index of (0.195 ± 0.012) and a surface potential of about (- 48.6 ± 1.3) mV. Colloidal stability of SPION/HA-FA nanoparticles was confirmed based on DLS measurements under storage conditions, i.e. at 4°C (stable for up to 10 weeks; *Figure 2A*), in physiological conditions (37°C, pH = 7.4) (stable for up to 50 hours; *Figure 2B*) and at pH imitating the tumor tissue environment (37°C, pH = 5.0) (stable for up to 16 hours; *Figure 2B*)*.*

The successive coupling of FA with the surface of SPION/HA-CYS is confirmed by the ATR-FTIR spectroscopic measurement (*Figure 1*), which shows bands characteristic of the formation of an amide bond: amide I at 1642 cm⁻¹ (stretching vibrations of C=O and C-N groups) and amide II at 1548 cm⁻¹ (N-H bending) and a band at 838 cm⁻¹ (aryl-H deformation vibrations in the aromatic ring from the FA structure). The amount of FA on the surface of SPION/HA-FA was assessed using UV-VIS spectrophotometry (*Figure 3*), and the FA content (expressed as a percentage) on the surface of SPION/HA-FA was estimated to be (28.5 ± 2.9) % based on the performed analysis.

Based on AFM, the average diameter of partially swollen SPION/HA-FA was determined to be (26 ± 4) nm, and the diameter of dry nanoparticles determined from TEM measurements was in the range of 5-10 nm. Because the polymeric HA layer swells very strongly in water (even a hundred times), such a difference is expected. Nanoparticles form small stable aggregates that resemble bead chains. The images are shown in *Figure 4**.*

### Structural, magnetic and dynamic characteristics of magnetic contrast agent nanoparticles

The structure of magnetic cores of the nanoparticles and sizes of crystallites were determined by X-ray diffraction. *Figure 5A* shows a diffractogram of the tested SPION and - for comparison - of microcrystalline magnetite.

It shows that the nanoparticles have a magnetite-like crystalline structure.. The broad lines of the SPION diffractogram indicate their nanometric sizes. Analysis using the Williamson-Hall method allowed the size of crystallites (magnetic cores) of nanoparticles to be determined from the line width plot (*Fig. 5B*) as 7 nm. The positions of the SPION lines observed at higher angles than for magnetite correspond to smaller lattice constants, which indicates the oxidized form of magnetite (maghemite) as the building material of SPION.

Magnetic properties were tested using vibrating sample magnetometry in a Quantum Design Physical Property Measurement System (PPMS) device equipped with a 9-Tesla superconducting magnet. *Figure 6A* shows magnetization curves - hysteresis loops for selected temperatures. They show non-zero coercivity (half loop width) at temperatures below 150 K (zoom - Figure *6B*). At 150 K and above, the coercivity field is zero, indicating a superparamagnetic state of SPION within this temperature range.

Figure 6C shows the temperature dependencies of low-field magnetic susceptibility (100 Oe) measured after cooling without field (ZFC) and in 100 Oe field (FC). They are characteristic of superparamagnetic materials, and the maximum of the ZFC curve, which corresponds to the superparamagnetic blocking temperature above which the magnetic moments of the nanoparticles fluctuate, is approx. 100 K.

The magnetic dynamics of SPION at room temperature was examined by ⁵⁷Fe Moessbauer spectroscopy (*Figure 6D*)*.*

The resulting spectrum has the shape of a funnel, in the bottom of which two lines are seen. This is characteristic of the relaxation spectrum of fluctuating nanoparticles. The ⁵⁷Fe spectrum for static magnetic moments consists of one sextet or superposition of sextets. From the theoretical fitting of the spectrum, the frequency of fluctuations of magnetic moments of the SPION nanoparticles was determined to be (123 ± 43.0) MHz.

¹H NMR relaxation coefficients were determined from measurements of T₁ and T₂ relaxation times for HA/SPION and HA/SPION-FA suspensions using a PS15 spectro-relaxometer operating at a frequency of 15 MHz (0.35 T field). The CPMG (Carr-Purcell-Meiboom-Gill) sequence was used to determine T₂ and the IR (Inversion-Recovery) sequence was used to determine T₁, from which relationships between concentration and 1/ T₁ and 1/ T₂ relaxation rates were determined.

Relaxivity r₁(T₁) was determined on the basis of linear approximation of the relationship between the relaxation rate, 1/T₁ and the iron content (present in the form of SPION) in suspensions, as slopes of fitted straight lines (Fig. 7A). For non-functionalized and FA functionalized nanoparticles, the values are 40 L/(mmol s) and 78 L/(mmol s), respectively. They are greater compared to those for commercially available contrast agents and compared to the SPIONs described by Szpak et al. [1], which indicates significantly higher efficiency of the obtained SPION/HA and SPION/HA-FA as T₁ contrast agents for scanner frequencies of approximately 15 MHz (*Figure 7A*)*.*

*Figure 7B* shows the concentration dependence of the 1/T₂ relaxation rate and it can be seen that the slope (r₂ (T₂)) for FA-functionalized nanoparticles, of 358 L/(mmol s), is more than twice the corresponding value for non-functionalized nanoparticles (139 L/(mmol s)). This shows that the effectiveness of SPION/HA-FA as a T₂ contrast agent will be significantly greater compared to SPION/HA and other cited agents. Different values of r₁/r₂ ratios for functionalized FA and non-functionalized SPION/HA indicate a significant difference in the fluctuation spectra between SPION/HA and SPION/HA-FA, responsible for the ¹H spin-spin relaxation processes. Magnetic resonance imaging (MRI) was performed on phantoms constructed in the form of a matrix of Eppendorf tubes (internal diameter: 6 mm) filled with suspensions of different concentrations of SPION/HA or SPION/HA-FA in 2% agarose gel. For a comparison, SPION/HA and SPION/HA-FA water suspensions of the same concentrations were tested, as well as water and agarose gel samples without SPION. The T₁ and T₂ weighted images obtained for the cross section of the phantoms are shown in Figure 8.

The measurements showed high efficiency of SPION/HA-FA as a contrast agent in both T1- and T2-weighted scans.". The T₁ weighted scan (Figure 8A) shows almost no signal for the water and gel reference samples, while the signal returns significantly at the lowest concentration used, 0.05 mM Fe for SPION/HA and SPION/HA-FA in water and gel. For the T₂ weighted scan (*Figure 8B*), the signal decreases with increasing concentration for SPION/HA-FA samples in gel and water faster than for the corresponding SPION/HA samples. As the field in the scanner (0.60 T) is significantly higher than in the relaxometer (0.35 T), a direct comparison of signal intensities between the sample sets, and reating them to the T₁ and T₂ relaxometric data would not be conclusive. Nevertheless, the results obtained for phantoms confirm the high efficiency of the produced SPIONs as contrast agents for both T₁ and T₂ relaxation times in low magnetic field scanners, confirming their potential in the MRI imaging of human tissues.

### Example 3. Characteristics of the biological properties of the resulting nanoparticles

Considering the planned intravenous administration of SPION/HA-FA, they will first interact with erythrocytes and leukocytes. Therefore, it is important to exclude the toxicity of nanoparticles to blood cells. The tested SPION/HA-FA does not cause hemolysis or changes in the morphology of human erythrocytes (*Figure 9*)*.* In addition, the results presented in *Figure 10* indicate that SPION/HA-FA does not affect the viability of human mononuclear cells (PBMCs) and neutrophils (PMNs) isolated from human peripheral blood. Thus, it was confirmed that SPION/HA-FA nanoparticles show biocompatibility with the cellular elements of human peripheral blood.

In addition, the toxicity of SPION/HA-FA to liver cells (HepG2), which are particularly exposed to xenobiotics, was assessed. The MTT test showed that SPION/HA-FA did not significantly reduce hepatocyte viability (*Figure 10*)*.* The above analyses confirm that SPION/HA-FA is safe for use in humans.

The resulting SPION/HA-FA is also able to actively target tumor cells overexpressing folic acid receptors, as confirmed by in *vitro* studies on two human cell lines: the A172 human glioblastoma multiforme cell line and the SW620 colon adenocarcinoma cell line.

Based on the long-term MTT test (24, 48 and 72 hours), a significant dose-dependent decrease in the viability of A172 glioblastoma multiforme cells after incubation with SPION/HA-FA nanoparticles (*Figure 11B*) was observed as early as 24 hours after nanoparticle administration.

Therefore, it is possible to use SPION/HA-FA as a contrast agent in the MRI imaging of tumors, with additional therapeutic benefit. Confocal microscopy imaging showed that SPION/HA-FA was effectively taken up by the A172 glioblastoma tumor cells (*Figure 12*)*.*

After 6-hour incubation of the A172 cells with SPION/HA-FA, a green fluorescence from FITC attached to the nanoparticle surface was observed entirely inside the cells, within the cytoskeleton (F-actin filaments). This confirmed the effective uptake of nanoparticles by the studied glioblastoma cells, i.e. the effective targeting of SPION/HA-FA to cancer cells with overexpressing foliate receptors.

### Conclusions:

**1.** The resulting nanoparticles exhibit all the features required of an MRI contrast agent with respect to:
   - size (less than 200 nm),
   - stability in the physiological environment (50 hours),
   - storage stability (10 weeks),
   - biocompatibility and safety of use.
**2.** The resulting particles exhibit unexpected magnetic properties enabling their use as an effective dual contrast agent (T₁ - T₂), with the performance superior to that of commercially available contrast agents ijn a specific mechanism of action, i.e. T₁ or T₂. The contrast agent of the invention is very effective for both T₁ weighted imaging (without the use of harmful paramagnetic ions such as Gd) and T₂ weighted imaging. Consequently, this allows for increased sensitivity in MRI examinations and a reduction in scan time..

According to Figure 7A, SPION/HA-FA has twice the T₁ relaxivity than SPION/HA, which at the same concentrations gives twice the signal in sites where SPION/HA-FA is present than in the case of SPION/HA. This is due to the twofold shortening of the proton relaxation time T₁ by SPION/HA-FA.. This facilitates, among other things, significant reduction of the repetition time of the measurement sequence when using SPION/HA-FA compared to that required for SPION/HA. Therefore, for example, a much larger number of repetitions of the sequence is possible at the same time interval, and thus a much better signal-to-noise ratio can be obtained. Furthermore, a smaller number of repetitions of the sequence is sufficient to obtain the same signal-to-noise ratio. Thus, performing a T₁ contrast examination using SPION/HA-FA will take much less time (approximately twice as short) than when using SPION/HA.
**3.** Due to the presence of both hyaluronic and folic acids on the surface, nanoparticles also doubly target cancer cells that overexpress the relevant receptors, i.e. CD44 (HA receptor) and FOLR1 (FA receptor), which further significantly increases contrast agent specyfic sensitivity in cancer imaging.

The significant increase in contrast sensitivity results both from the previously described increase in the signal-to-noise ratio in the case of SPION/HA-FA and from the selective interaction of these nanoparticles with cancer cells. The difference in this interaction cannot be unequivocally estimated, because it will depend on the type of tumor, but it is illustrated using the A172 glioblastoma multiforme cell line (malignant brain tumor) as an example. Figure 11 related to the MTT test shows that on the first day, the viability of cancer cells changes clearly with a very small amount of SPION/HA-FA nanoparticles present (1 µg/mL), and for the SPION/HA system, the change is observed only at a concentration 50 times higher (50 µg/mL). This effect is associated with overexpression (significantly higher than in healthy cells) of the folic acid (FA) receptor on the surface of these cells (this is quite common in various types of tumors). As a result, SPION/HA-FA nanoparticles penetrate cells much more effectively than SPION/HA. SPION/HA-FA selectivity against tumor cells will allow for a significantly (at least several fold) higher accumulation of SPION/HA-FA at the tumor site compared to SPION/HA, resulting in a substantially greater T₁ contrast when using SPION/HA-FA. The contrast will be further enhanced by the twofold higher relaxivity of SPION/HA-FA, resulting in a significantly stronger signal when using the same measurement sequence.

In an embodiment, SPION/HA-FA T₁ relaxivity was twice as high as that of SPION/HA.

More selective accumulation of SPION/HA-FA in the tumor tissue relative to SPION/HA has been demonstrated based on their effect on glioblastoma multiforme cells (Figure 11) and it is associated with the presence of FA as a targeting unit.

Owing to the aforementioned twice as high T₁ relaxivity of SPION/HA-FA, the examination time can be reduced approx. two-fold compared to SPION/HA or sensitivity can be increased approximately as much. The effect of SPION/HA-FA affinity to tumor cells will depend on the type and location of the tumor; however, it can be expected that it may enable an additional several-fold reduction in measurement time or a several-fold increase in sensitivity.

## Claims

1. A contrast agent (SPION/HA-FA) consisting of superparamagnetic iron oxide nanoparticles (SPION) surface-stabilized with low-molecular weight hyaluronic acid (HA) with covalently attached folic acid (FA).

2. The contrast agent of Claim 1, **characterized in that** it has a form of nanoparticles with hydrodynamic diameter below 200 nm and showing stability in the physiological environment for at least 50 hours.

3. A process for the preparation of the contrast agent, **characterized in that**:
a) superparamagnetic iron oxide nanoparticles stabilized with low-molecular weight hyaluronic acid (SPION/HA) are obtained,
b) the surface of SPION/HA obtained in step a) is modified by covalently attaching folic acid molecules, and subsequently
c) the superparamagnetic iron oxide nanoparticles surface-stabilized with low-molecular weight hyaluronic acid with covalently attached folic acid (SPION/HA-FA) obtained in step b) are separated.

4. The process of Claim 3, **characterized in that** in step a) a solution of hyaluronic acid sodium salt in ammonia water is pretreated by stirring for at least 18 hours at room temperature and pulse sonication for 2.5-4 hours at not less than 50°C, subsequently the temperature of the solution is set at 60°C and in the presence of an inert gas, iron(III) chloride and iron(ll) chloride are added, preferably in a molar ratio of 2:1, followed by an ammonia water solution, preferably in an amount that achieves a concentration in the reaction mixture in the range of 1.5-2.0 M, and then it is left to obtain a colloidal suspension of SPION/HA, which is separated.

5. The process of Claim 3, **characterized in that** in step b), SPION/HA modified with cystamine dichloride (SPION/HA-CYS) is first obtained by conducting the SPION/HA reaction in the presence of N-(3-dimethylaminopropyl)-N'-ethylcarbodiimide (EDC) and N-hydroxysuccinimide (NHS), preferably in a 1:1 ratio, continuing the reaction for 15-24 hours at 37°C with shaking, and then the thus obtained SPION/HA-CYS is separated and reacted with folic acid dissolved in anhydrous dimethyl sulfoxide (DMSO) in the presence of EDC, NHS, preferably in a 1:1 ratio, and triethylamine (TEA) as a catalyst, without light, previously bringing the pH of the SPION/HA-CYS suspension to the range of 9.5-10.

6. The process of Claim 3, **characterized in that** in step c) dialysis against deionized water is carried out, preferably using a membrane having an MWCO of 14 kDa.
